# EUROPÄISCHE PATENTSCHRIFT

(11) **EP 0 599 159 B1**
(45) Veröffentlichungstag und Bekanntmachung des Hinweises auf die Patenterteilung: **19.09.2001**
(21) Anmeldenummer: 93118383.4
(22) Anmeldetag: 12.11.1993
(51) Int. Cl.: C12N 9/24, C12P 19/14, C12N 1/14

(54) **Heterogenes Proteingemisch mit alpha-L-rhamnosidase Aktivität, Verfahren zu ihrer Herstellung und ihre Verwendung.**
Heterogeneous protein mixture with alpha-L-rhamnosidase activity, process for preparation and use.
Mélange de protéines hétérogènes, avec une activité alpha-L-rhamnosidase, procédé de préparation et d'utilisation.

(30) Priorität: 27.11.1992 DE 4239859
(43) Veröffentlichungstag der Anmeldung: 01.06.1994
(73) Patentinhaber: Aventis Research & Technologies GmbH & Co. KG, 65926 Frankfurt am Main (DE)
(72) Erfinder: Meiwes, Johannes, Dr., D-65510 Idstein (DE); Wullbrandt, Dieter, Dr., D-65719 Hofheim/Taunus (DE); Giani, Carlo, Dr., D-60596 Frankfurt/Main (DE)
(74) Vertreter: Ackermann, Joachim, Dr.

(56) Entgegenhaltungen:
- EP-A- 0 317 033
- HOPPE SEYLER Z. PHYSIOL. CHEM., Bd. 365,Nr. 9, 1984 Seite 914 GABOR ET AL. 'Daten zur Karakterisierung von naringinase aus Penicillium species'
- CHEMICAL ABSTRACTS, vol. 98, no. 19, Mai 1983 Columbus, Ohio, US; abstract no. 159152w, 'Manufacture of thermostable hesperidinase AH-2' Seite 387; Spalte 159153; & JP-A-58 020 189 (AMANO PHARMACEUTICAL CO LTD) 5.Februar 1983

## Beschreibung

Die Erfindung betrifft ein heterogenes Proteingemisch mit der Aktivität einer α-L-Rhamnosidase welche die Spaltung der Bindung zwischen terminaler L(+)-Rhamnose und Aglykon von rhamnosehaltigen Glykosiden katalysiert, ein Verfahren zur biotechnischen Herstellung und ihre Verwendung für die Herstellung von L(+)-Rhamnose (6-Desoxy-L-Mannose). Dieser letztgenannte Zucker wird im folgenden als L-Rhamnose bezeichnet.

L-Rhamnose eignet sich als chiraler Baustein sehr gut zur Herstellung verschiedener organischer Verbindungen. L-Rhamnose oder deren Derivate finden immer breitere Anwendung bei der Synthese von pharmazeutischen Erzeugnissen und Pflanzenschutzmitteln, wie auch im Bereich der Cytologie pflanzlicher und tierischer Zellen, der Mikrobiologie, Immunobiologie und Aromaherstellung. So läßt sich z.B. mit L-Rhamnose als Ausgangsverbindung 2,5-Dimethyl-4-hydroxy-2,3-dihydrofuran-3-on (Furaneol®) herstellen, das wiederum als Grundkörper verschiedener Aromastoffe in der Nahrungsmittel- und Duftstoffindustrie dient.

L-Rhamnose ist auf chemischem Weg nur sehr schwer zugänglich. Er läßt sich aber aus verschiedenen, natürlichen Quellen extraktiv nach saurer Hydrolyse gewinnen, so z.B. aus den Flavonglykosiden Hesperidin, Rutin, Naringin, Quercitrin oder z.B. aus Gummi arabicum oder Meeresalgen. [(Biotechnology and Bioengineering, Vol. 33, S. 365 (1989), R. J. Linhardt et al.; EP-A-0 317 033; JPA 62293]. Nachteilig für diese Verfahren wirken sich die aufwendigen Isolierungsschritte für L-Rhamnose, teilweise unter Verwendung organischer Lösungsmittel, ferner die bei der Aufarbeitung anfallenden aromatischen, potentiell toxischen Abfallprodukte und die in der Zusammensetzung schwankenden, vom jahreszeitlichen Rhythmus abhängigen Inhaltsstoffe der natürlichen Quellen aus.

Fermentativ läßt sich L-Rhamnose auch in Form rhamnoseenthaltender Heteropolysaccharide mit Hilfe von Bakterien verschiedener Gattungen wie z. B. Alcaligenes, Acinetobacter, Klebsiella, Streptococcus oder Lactobacillus herstellen.
[Enzyme Microb. Technol., Vol. 10, S. 198 (1988), M. Graber et al.; J. Amer. Chem. Soc., Vol. 71, S. 4124 (1945), F. G. Jarvis und M. J. Johnson; J. Bacteriol., Vol. 68, S. 645 (1954), G. Hauser and M. L. Karnovsky].

Nachteilig bei diesen Verfahren sind die üblicherweise viskositätsbedingt geringen Ausbeuten und die nach hydrolytischer Spaltung des Heteropolysaccharides notwendige Trennung der L-Rhamnose aus einem Gemisch verschiedener Zucker.

Zahlreiche Veröffentlichungen und Patente befassen sich mit der fermentativen Gewinnung von Rhamnolipiden durch Pseudomonas aeruginosa. [Applied and Environmental Microbiology, Vol. 51, No. 5, S. 985 (1986), H. E. Reiling et al.; J. Chem. Techn. Biotechnol., Vol. 45, S. 249 (1989), K. Venkata Ramana et al.; US-Patent 4 933 281, Daniels et al.; Deutsche Offenlegungsschrift 2 150 375, 1972; US-Patent 4 814 272, Wagner et al.]

In der Kulturlösung von Mikroorganismen kommen grundsätzlich 4 Rhamnolipide (RL1 - RL4, siehe Abb. 1) vor, die aus 1 oder 2 L(+)-Rhamnose-Einheiten und ein oder zwei β-Hydroxydecansäuren bestehen [Z. Naturforsch. 40 c, S. 61 (1985), C. Syldatk et al.]. Quantitativ den größten Anteil bilden die Rhamnolipide 1 und 3.

Abbildung 1: Rhamnolipide aus Mikroorganismen

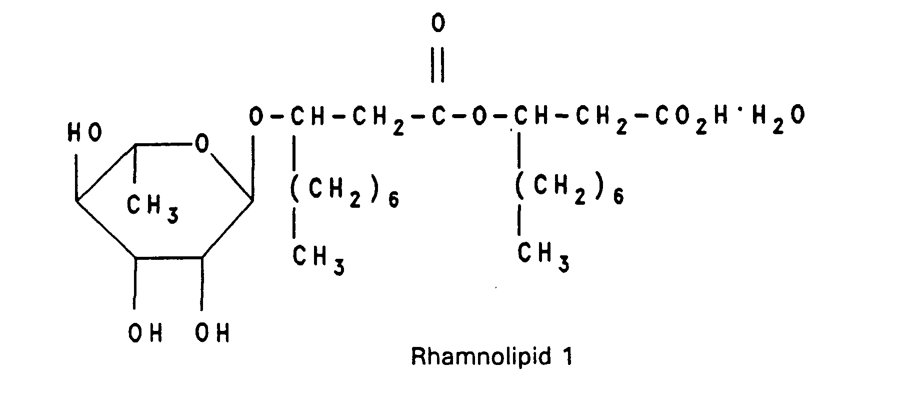

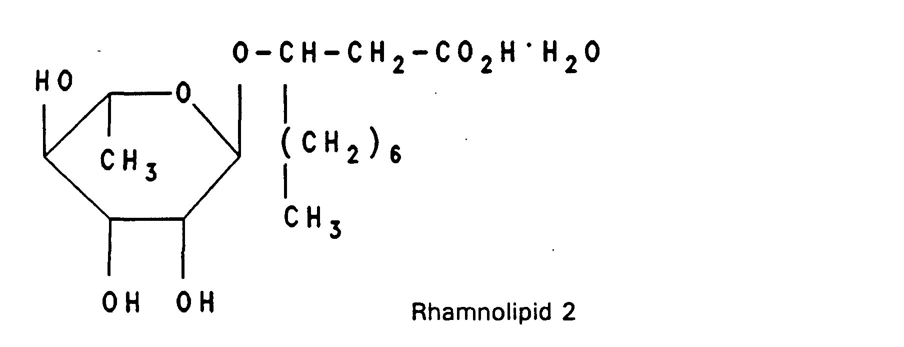

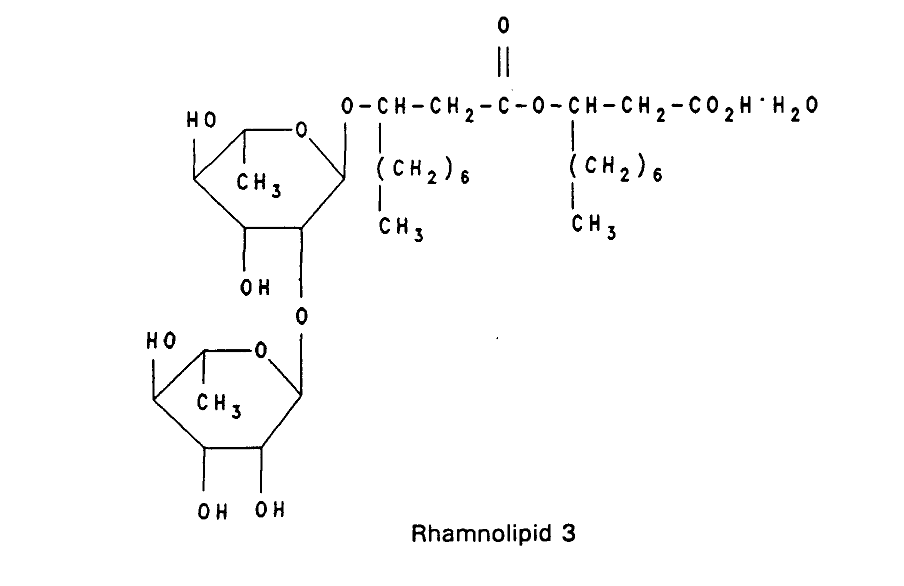

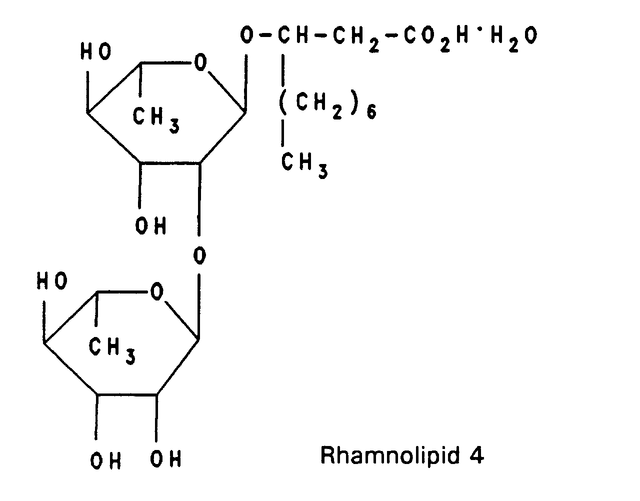

Außerdem bekannt ist die Gewinnung von L-Rhamnose aus Flavonglycosiden, Rhamnolipiden oder Oligosacchariden durch enzymatische Spaltung mit den löslichen oder immobilisierten α-L-Rhamnosidasen Naringinase und Hesperidinase [US Pat. 5,077,206; Eur. Pat. 88202595.0; Turecek, P. und Pittner, F., Appl. Biochem. und Biotech. 13, 1-13 (1986)]. Naringinase und Hesperidinase haben jeweils ein Molekulargewicht von ca. 90 kd und wurden aus Penicillium decumbens bzw. Aspergillus niger isoliert.

Naringinase und Hesperidinase katalysieren die Spaltung der Bindung zwischen zwei Monosacchariden, hauptsächlich die Abspaltung von terminaler Rhamnose aus Flavanonglykosiden, wie z.B. aus Hesperidin, Naringin oder aus Rhamnolipid 3 bzw. 4.

Die Katalyse der Bindungsspaltung zwischen terminaler Rhamnose und einem Aglykon von rhamnosehaltigen Glykosiden durch Nariginase und Hesperidinase erfolgt deutlich langsamer (Faktor: 10-100; s. Beispiel 1).

Dies führt dazu, daß zur kompletten Abspaltung der Rhamnose aus dem Gemisch Rhamnolipid 1-4 ein sehr langer Zeitraum notwendig ist, da das quantitativ in der Kulturlösung von Mikroorganismen am meisten vorhandene Rhamnolipid 1 und 3 aus L-Rhamnose und Aglykon (Fettsäure) bestehen.

Es wurde nun überraschend aus Penicillium sp. ein heterogenes Proteingemisch mit der Aktivität einer α-L-Rhamnosidase isoliert, welches die Spaltung der Bindung zwischen terminaler L-Rhamnose und Aglycon von rhamnosehaltigen Glykosiden katalysiert, also die umgekehrte Spezifität im Vergleich zu den bekannten α-L-Rhamnosidasen Nariginase und Hesperidinase besitzt.

Die Erfindung betrifft somit
1. ein heterogenes Proteingemisch mit der Aktivität einer α-L-Rhamnosidase, erhältlich
   - durch Fermentation von Penicillium sp. DSM 6825 und/oder 6826
   - Abtrennung der Biomasse von der Kulturbrühe und
   - Konzentrierung des Kulturüberstands.
2. ein heterogenes Proteingemisch mit der Aktivität einer α-L-Rhamnosidase und zwar mit einem Molekulargewicht von 60 - 100 kd und den aminoterminalen Aminosäuresequenzen D-T-N-D-Q-T-S-A-K-V-D-R-G-T-F-D-D-P-A-A-R-L oder F-F-G-S-X-Q-S-L-Y-L-K-L-V-L-K-F-G-T-L-F-D-X-A welche die Spaltung der Bindung zwischen terminalen L-Rhamnose und Aglykon von rhamnosehaltigen Glykosiden katalysiert.
3. Ein Verfahren zur Herstellung eines solchen heterogenen Proteingemisches mit der Aktivität einer α-L-Rhamnosidase, dadurch gekennzeichnet, daß Penicillium sp. in einem Nährmedium kultiviert wird, wobei sich ein heterogenes Proteingemisch mit der Aktivität einer α-L-Rhamnosidase in der Kultur anhäuft, die Biomasse von der Kulturbrühe abgetrennt und der so erhaltene Kulturüberstand aufkonzentriert wird.
4. Verwendung eines solchen heterogenen Proteingemisches mit der Aktivität einer α-L-Rhamnosidase zur Herstellung von L-Rhamnose.
5. Penicillium sp. DSM 6825
6. Penicillium sp. DSM 6826

Die Erfindung wird im folgenden detailliert beschrieben, insbesondere in ihren bevorzugten Ausführungsformen.

Als L-Rhamnose wird die Verbindung L(+)-Rhamnose (= 6-Desoxy-L-Mannose) bezeichnet.

Als Aglykon wird eine Verbindung oder der Anteil einer Verbindung bezeichnet, der keinen Zucker enthält. Bei dieser Erfindung werden als Aglykon insbesondere Fettsäure - oder Flavonverbindungen bezeichnet.

Für die Aminosäuren werden folgende Abkürzungen verwendet, die dem aus der Fachliteratur bekannten "Single-letter-code" entsprechen.

Das heterogene Proteingemisch mit der Aktivität einer α-L-Rhamnosidase kann aus der Kulturbrühe sowohl in kleinen Mengen (Mengen bis zu 1 Gramm) wie auch in großen Mengen (≤ 1 kg) gewonnen werden, da das Verfahren zur Herstellung im Labormaßstab (Fermentation der Mikroorganismen in Volumina bis zu 1 Liter) und im industriellen Maßstab (Fermentation der Mikroorganismen im Kubikmetermaßstab) durchgeführt werden kann.

Das Molekulargewicht des erfindungsgemäßen heterogenen Proteingemisches mit der Aktivität einer α-L-Rhamnosidase wird mit der SDS-Gelelektrophorese (SDS = Sodium Dodecylsulfat) sowie der Gelchromatographie bestimmt. Diese Methode der Gelchromatographie ist beispielsweise beschrieben in "Molecular Biology of the Cell", Bruce Alberts et al., Garland Publishing, Inc. New York & London, 3. Auflage, 1983, S. 174, 265 - 266.

Die Abkürzung "IEP" steht für "Isoelektrischer Punkt" und ist definiert als der pH-Wert, an dem die Nettoladung des Proteins bzw. im vorliegenden Fall des Enzyms, Null ist. Der IEP wird mit Hilfe der Chromatofokussierung bestimmt.

Penicillium sp. wurde aus einem Kompost aus Gartenabfällen in Bad Soden, Hessen, Deutschland, isoliert. Die Isolierung und Aufreinigung des Mikroorganismus erfolgte nach dem Fachmann bekannten Verfahren durch Kulturverdünnung und Ausplattieren auf Selektivagar. Beispielsweise kann die Kompostprobe in 0,9 %iger Kochsalzlösung suspendiert und von dieser Suspension eine Anreicherungskultur in Selektivmedium mit Rhamnolipiden und/oder Rhamnolipidderivaten, vorzugsweise Rhamnolipid-2-C₁-C₁₈-Alkylester als einziger Kohlenstoffquelle angelegt werden. Besonders bevorzugt werden als C-Quelle Rhamnolipid -2-C₁-C₄-Alkylester, wie z.B. Rhamnolipid-2-Methylester oder Rhamnolipid-2-tert.-Butylester verwendet.

Formeln 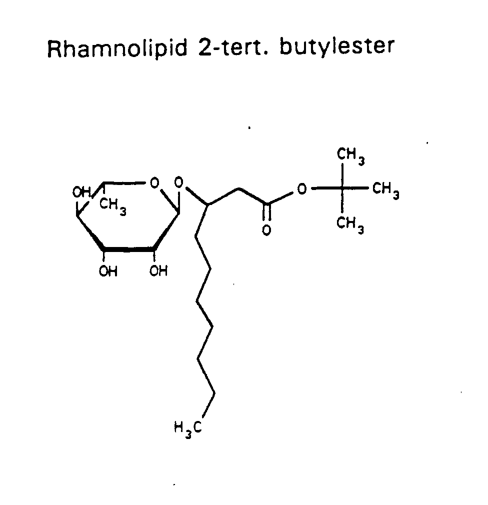 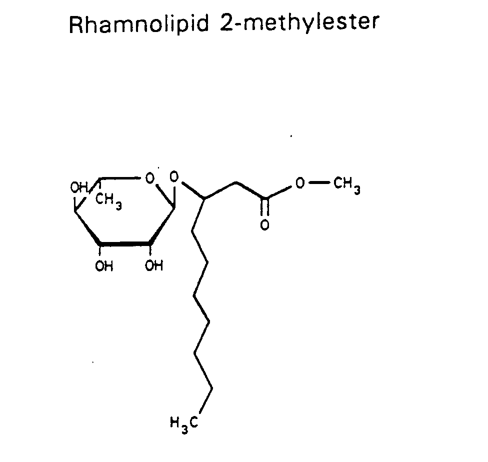 Penicillium sp. DSM 6825 und DSM 6826 haben folgende morphologische Charakteristika (nach R.A. Samson et al., Introduction to Food-borne Fungi, Institute of the Royal Netherlands Academy of Arts and Sciences, 3. Auflage, 1988):

| Penicillium sp. DSM 6825 | |
|---|---|
| Verzweigung der Konidien | monoverticilliat |
| Phialiden | ampulli-form |
| Konidien | stachelig |

| Penicillium sp. DSM 6826 | |
|---|---|
| Verzweigung der Konidien | biverticilliat |
| Phialiden | flaschenförmig |
| Konidien | warzig |

Penicillium sp. DSM 6825 und 6826 können gemeinsam oder getrennt fermentiert werden.

Anstelle der Isolate DSM 6825 und/oder 6826 können auch Mutanten und Varianten eingesetzt werden, soweit sie das Enzym α-L-Rhamnosidase produzieren. Solche Mutanten können in an sich bekannter Weise durch physikalische Mittel, beispielsweise Bestrahlung, wie mit Ultraviolett- oder Röntgenstrahlen oder chemische Mutagene, wie z.B. Ethyl-methansulfonat (EMS),2-Hydroxy-4-methoxy-benzophenon (MOB) oder N-Methyl-N'-nitro-N-nitrosoguanidin (MNNG) erzeugt werden.

Das Verfahren zur Herstellung des oben genannten heterogenen Proteingemisches mit der Aktivität einer α-L-Rhamnosidase ist wie folgt:

Nachfolgend zur Isolierung und Aufreinigung von Penicillium sp. (s. S. 6) durch mehrfache Passage der Mischkultur der entstandenen Mikroorganismen in Selektivmedium wird eine Anreicherung der Mikroorganismen, die das erfindundsgemäße heterogene Proteingemisch produzieren, erreicht.

Die so erhaltenen Mikroorganismen werden auf Agarplatten (Selektivmedium) ausplattiert, um aus der Mischkultur Reinkulturen zu erhalten.

Die Reinkulturen werden vermehrt und auf ihre Fähigkeit zur Bildung des erfindungsgemäßen heterogenen Proteingemisches getestet.

Es zeigt sich, daß Mikroorganismen der Gattung Penicillium sp. das erfindungsgemäße Enzym bilden. Die Bestimmung der Pilzgattung erfolgt anhand von morphologischen, taxonomischen und biochemischen Kriterien nach dem Fachmann bekannten Methoden. Die Koloniefarbe ist grün.

Die Untersuchungen der Fähigkeit der Penicillium sp. Kolonien zur Bildung des erfindungsgemäßen heterogenen Proteingemisches führen zur Isolierung von zwei Stämmen, die sich durch eine besonders hohe Produktion von α-L-Rhamnosidase auszeichnen. Diese beiden Stämme sind: Penicillium sp. DSM 6825 und DSM 6826.

Die Stämme wurden bei der Deutschen Sammlung von Mikroorganismen und Zellkulturen GmbH, Mascheroder Weg 1B, Braunschweig, Hessen, Deutschland, nach den Regeln des Budapester Vertrages am 29. November 1991 unter den Nummern: Penicillium sp. DSM 6825 und 6826 hinterlegt.

Die Kultivierung der betreffenden Mikroorganismen erfolgt unter den für Penicillium sp. üblichen Bedingungen. Demzufolge erfolgt die Anzucht auf Komplex- oder definierten Medien, vorzugsweise enthalten die Medien

Hefeextrakt, Casamino acids, Cornsteep, Fleischextrakt, Pepton, Casein, Gelatine, Trypton, Nitrat, Ammonium oder Harnstoff als Stickstoffquelle und Stärke, Dextrin, Saccharose, Glucose, Glycerin, Malzextrakt als Kohlenstoffquelle.

Als weitere Komponenten können Magnesium, Calcium, Natrium, Kalium, Eisen, Zink, Kobalt oder Phosphat eingesetzt werden. Als besonders bevorzugt erweist sich die Verwendung von Rhamnolipiden oder deren Alkylester (Rohgemische oder gereinigte Rhamnolipide) als C-Quelle, allein oder in Kombination mit weiteren C-Quellen.

Die Kultivierung erfolgt bei 27°C über 72 - 120 Std. Die gegebenenfalls nötige Isolierung der erfindungsgemäßen α-L-Rhamnosidase erfolgt auf übliche Weise, z.B. durch Filtration oder Zentrifugation zur Abtrennung der Biomasse, wobei die α-L-Rhamnosidase sich größtenteils im Überstand befindet.

Der Überstand kann durch Ultrafiltration konzentriert oder lyophilisiert werden. Weitere Reinigungsschritte wie z.B. Fällungen, Anionenaustausch-Chromatographie, Chromatofokussierung HIC-Chromatographie (Hydrophobic Interaction Chromatography), Ausschluß-Chromatographie sowie Affinitäts-Chromatographie können je nach gewünschter Reinheit des Enzyms durchgeführt werden.

Vorzugsweise erfolgt die Aufreinigung durch Filtration zur Abtrennung der Biomasse, nachfolgender Ultrafiltration zur Konzentration des Enzyms in dem verbleibenden Überstand, anschließenden Anionenaustauscherchromatographie, Chromatofokussierung und zuletzt die Ausschlußchromatographie.

Das heterogene Proteingemisch mit der Aktivität einer α-L-Rhamnosidase besitzt ein Molekulargewicht von 60 - 100 kD in Abhängigkeit vom Glykosilierungsgrad und einen isoelektrischen Punkt von 5,6 - 5,8. Das pH-Optimum der Spaltung von p-Nitrophenyl-α-L-Rhamnopyrasid liegt bei 5,0 - 5,5 das Temperaturoptimum bei 50 - 55°C.

Die Bestimmung der aminoterminalen Sequenz aus dem Stamm Penicillium sp. DSM 6826 und der Naringinase erfolgt grundsätzlich gemäß dem literaturbekannten Edman-Verfahren. Hierzu werden Aminosäure-Ketten unter geeigneten Bedingungen mit Phenylisothiocyanat gemischt. Die chemische Verbindung lagert sich bevorzugt an die freie aminoterminale Amino-Gruppe. In Gegenwart von wasserfreier Säure wird die endständige Aminosäure als ein Carbamyl-Derivat abgespalten. Diese Verbindung wird zur Identifizierung der aminoterminalen Aminosäure untersucht. Der Rest der Aminosäure-Kette (dem jetzt die ursprüngliche Aminosäure fehlt) kann nun einer erneuten Behandlung mit Phenylisothiocyanat unterworfen werden zur Bestimmung der nächstfolgenden Aminosäure usw. Im Prinzip kann man diesen Arbeitsvorgang mehrfach schrittweise hintereinander durchführen, so daß die gesamte Aminosäure-Frequenz der Kette abgeleitet werden kann. Im vorliegenden Fall wird außerdem die N-terminale Aminosäuresequenzierung mit einem Gasphasensequencer (Typ 477 A von Fa. Applied Biosystems) und die Aminosäurenanalysemit einem Online-Aminosäurenanalysator (Typ 130 A PTC-Analyzer von Fa. Applied Biosystems) durchgeführt. Die Methoden sind publiziert in FEBS Letters, Vol. 292, S. 405- 409, 1991.

Für das heterogene Proteingemisch mit der Aktivität einer α-L-Rhamnosidase konnten folgende aminoterminale Teilsequenzen ermittelt werden: D-T-N-D-Q-T-S-A-K-V-D-R-G-T-F-D-D-P-A-A-R-L oder

F-F-G-S-X-Q-S-L-Y-L-K-L-V-L-K-F-G-T-L-F-D-X-A.

Das erfindungsgemäße heterogene Proteingemisch mit der Aktivität einer α-L-Rhamnosidase kann in freier oder immobilisierter Form eingesetzt werden, wobei hierzu alle gängigen Immobilisierungsmethoden in Frage kommen. Als Träger kann z.B. Kieselgel (aus Kostengründen) verwendet werden.

Das heterogene Proteingemisch mit der Aktivität einer α-L-Rhamnosidase katalysiert die Spaltung der Bindung zwischen terminaler L-Rhamnose und Aglykon von rhamnosehaltigen Glykosiden und eignet sich somit zur Herstellung von Rhamnose.

Die Spaltung wird in wäßrigen Lösungen, die gepuffert oder nicht-gepuffert sind, durchgeführt. Wäßrige Lösungen sind beispielsweise die Kulturbrühe des Mikroorganismus (keine Pufferung notwendig) oder destilliertes Wasser. Im letztgenannten Fall ist eine Pufferung mit Phosphat- oder Trispuffer, vorzugsweise Ammoniumacetatpuffer, notwendig (Konzentration des Puffers: 5-100 mM, vorzugsweise 10-50mM). Der pH-Wert der wäßrigen Lösung ist pH 3,5-8, vorzugsweise pH 5-6. Die für die Enzymaktivität notwendige Temperatur liegt zwischen 4°C-65°C, vorzugsweise 45°C-55°C. Die Reaktionszeit ist abhängig von der Enzym- und Substratmenge und geringfügig auch von der Temperatur. Bei einer Temperatur von 45°C-55°C beträgt die Reaktionszeit 2-24 Stunden, vorzugsweise 5-8 Stunden. Bei höheren Temperaturen ist eine kürzere Reaktionszeit sinnvoll, da das Enzym leichter degradiert. Die im Ansatz befindliche Substratmenge (= Menge an Rhamnolipiden) beträgt maximal 200 g/l, die Enzymmenge 0,1-50 U/gRhamnolipide, bevorzugt 1-10 U/g und besonders bevorzugt 5 U/g Rhamnolipide.

Nach Beendigung der Reaktion isoliert man die L-Rhamnose aus der Lösung durch Abtrennung der Fettphase mittels Zentrifugation oder Decanter bis eine Phasentrennung erfolgt ist, gegebenenfalls erfolgt nachfolgend eine Klärung der wäßrigen Phase z.B. mittels Aktivkohle. Unter Klärung versteht man das Entfernen von Trüb- und Farbstoffen. Dieser Schritt ist sinnvoll, wenn man eine möglichst reine L-Rhamnose erhalten soll. Abschließend erfolgt die Konzentration der wäßrigen Lösung und das Auskristallisieren der L-Rhamnose.

### Beispiel 1

### Spaltung der Rhamnolipide 1 und 3 durch Naringinase und Hesperidinase

10 g Rhamnolipid 1 oder 3 werden in 100ml Ammoniumacetatpuffer (50 mM, pH 5,5) oder Aqua bidest. emulgiert und mit 150 U Naringinase oder Hesperidinase (Fa. Sigma, Deutschland) versetzt. Die Umsetzung erfolgt bei 70°C unter Rührung. Die unter diesen Bedingungen erzielten Werte für vₘₐₓ sind in Tabelle 1 zusammengefaßt. Die Naringinase spaltet dabei in 4 Std. (Hesperidinase 7 Std.) die eingesetzten 10 g Rhamnolipid 3 in 2,5 g Rhamnose (∼ 98% Ausbeute) und Rhamnolipid1. Die Spaltung des Rhamnolipid 1 verläuft wesentlich langsamer (siehe Tabelle 1) und unvollständig, wahrscheinlich aufgrund der Inaktivierung des Enzyms über den relativ langen Zeitraum.

Die Umsetzung wurde mittels DC verfolgt, die quantitative Bestimmung der Rhamnose erfolgte mit HPLC.

**Tabelle 1:**

| | | |
|---|---|---|
| DC | Laufmittel | CHCl₃/CH₃OH/HAc 65:5:2 |
| | DC-Platte | Kieselgel 60 F254 |
| | Sprühreagenz | MeOH/HAc/H₂SO₄-konz./Anisaldehyd |
| | | 85:10:5:1 |
| | Entwicklung | 5 Minuten 120°C |

Enzymatische Spaltung von Rhamnolipid 1 und 3 mittels Hesperidinase und Naringinase [Die vₘₐₓ-Werte beziehen sich auf 1 U α-L-Rhamnosidaseaktivität; 1 U ist definiert als die Enzymmenge, die in der Lage ist, pro 1 *µ* Mol/Minute p-Nitrophenyl-α-L-Rhamnopyranosid zu spalten]:

| | | |
|---|---|---|
| Hesperidinase | (Sigma No. H-8137, Penicillium spec.) | |
| | "vₘₐₓ" Rhamnolipid 3: | _{~}50µgmn⁻¹u⁻¹ |
| | "vₘₐₓ" Rhamnolipid 1: | _{~}0,5µgmin⁻¹u⁻¹ |
| Naringinase | (Sigma No. N-1385, Penicillium decumbens) | |
| | "vₘₐₓ" Rhamnolipid 3: | _{~}80µgmin⁻¹u⁻¹ |
| | "vₘₐₓ" Rhamnolipid 1: | _{~}5,0µgmin⁻¹u⁻¹ |

| | | |
|---|---|---|
| HPLC | Säule | HPAP (100x7.8 mm) Biorad |
| | Vorsäul | Carbo P (30x4,6) Biorad |
| | Temperatur | 85°C |

| | |
|---|---|
| Eluent | Aqua bidest. |
| Fluß | 0,4 ml/Minute |
| Auftrag | 5µl |
| Detektor | Differential Refraktometer (Beckmann) |

Die Aufarbeitung der Rhamnose erfolgt nach den üblichen in der Literatur beschriebenen Methoden (PCT-EP 91-01426).

### Beispiel 2

### Spaltung der Rhamnolipide 1 und 3 durch das heterogene Proteingemisch mit der Aktivität einer α-L-Rhamnosidase aus Penicillium sp. DSM 6825 und/oder 6826.

10 g Rhamnolipid 1 oder 3 werden in 100 ml Ammoniumacetatpuffer (50 mM, pH 5,0) oder Aqua bidest emulgiert und mit 150 U des erfindungsgemäßen Enzyms versetzt. Die Umsetzung erfolgt bei 50°C unter Rühren. Die unter diesen Bedingungen erzielten Werte für vₘₐₓ sind in Tabelle 2 zusammengefaßt. Die erfindungsgemäße α-L-Rhamnosidase spaltet dabei in ca. 5-8 Std. (α-L-Rhamnosidase aus Penicillium sp. DSM 6825: ca. 8 Std.; α-L-Rhamnosidase aus Penicillium sp. DSM 6826: ca. 5 Std.) die eingesetzten 10 g Rhamnolipid 1 in 3,05 g Rhamnose (~ 94 % Ausbeute) und die entsprechenden Fettsäure. Die Spaltung des Rhamnolipid 3 verläuft wesentlich langsamer (siehe Tab. 2) und unvollständig, wahrscheinlich ebenfalls aufgrund der Inaktivierung des Enzyms über den relativ langen Zeitraum.

### Tabelle 2:

Enzymatische Spaltung der Rhamnolipide. Die vₘₐₓ.-Werte beziehen sich auf 1 U α-L-Rhamnosidaseaktivität; 1 U ist definiert als die Enzymmenge, die in der Lage ist, pro Minute 1 µMol p-Nitrophenyl-α-L-Rhamnopyranosid zu spalten:

| | | |
|---|---|---|
| α-L-Rhamnosidase | Penicillium sp. DSM 6825 | |
| aus Penicillium sp. 6825: | "vₘₐₓ" Rhamnolipid 3: | _{~}0,03µgmin⁻¹u⁻¹ |
| | "vₘₐₓ" Rhamnolipid 1: | _{~}40,0µgmin⁻¹u⁻¹ |
| α-L-Rhamnosidase | Penicillium sp. DSM 6826 | |
| aus Penicillium sp. 6826: | "vₘₐₓ" Rhamnolipid 3: | _{~}0,05µgmin1.⁻¹u⁻¹ |
| | "vₘₐₓ" Rhamnolipid 1: | _{~}70,0µgmin⁻¹u⁻¹ |

Die Isolierung der L-Rhamnose erfolgt wieder nach den bekannten Methoden, zusätzlich kann durch Extraktion im Sauren die abgespaltene Fettsäure isoliert werden.

### Beispiel 3

### Screening nach α-L-Rhamnosidase produzierenden Stämmen

Aus verschiedenen Bodenproben wurden Mikroorganismen auf Nährmedien mit Rhamnolipid-2-Methylester bzw. Rhamnolipid-2-tert.Butylester als einziger C-Quelle mit gängigen mikrobiologischen Methoden (Drews, Mikrobiologisches Praktikum, 45-84, Springer Verlag 1983) angereichert und Reinkulturen isoliert. Von den etwa 400 isolierten Stämmen waren rund 37 in der Lage Rhamnolipide abzubauen. Lediglich bei einem Teil der Stämme konnte jedoch eine signifikante α-L-Rhamnosidaseaktivität nachgewiesen werden; dabei erwiesen sich zwei Stämmen (Penicillium sp. DSM 6825 und Penicillium sp. DSM 6826) als besonders gute Produzenten.
Als Substrat für den Nachweis der α-L-Rhamnosidaseaktivität wird p-Nitrophenyl-α-L-Rhamnopyranosid verwendet. 10 mg dieses Substrates werden in 10 ml Ammoniumacetatpuffer (pH 5,5 50 mM) gelöst. 900*µ*l dieser Lösung wird mit 100 *µ*l Kulturfiltrat oder Zellysaten versetzt und bei 40°C inkubiert. Nach 0,3,6,9,12 Minuten werden 200 µl entnommen und mit 800 µl 200 mM Boratpuffer pH 9 vermischt. Die Abspaltung von p-Nitrophenol wird bei 410 nm photometrisch verfolgt, als Kontrolle diente Naringinase (angelehnt an Romero et al. Anal. Biochem. 149, 566-571 (1985).

### Beispiel 4

### Produktion der α-L-Rhamnosidasen - Aktivitäten mit den Stämmen Penicillium sp. DSM 6825 und Penicillium sp. DSM 6826.

Die Stämme werden zunächst auf Agarplatten mit HA-Medium (Hefeextrakt 4 g/l, Malzextrakt 10 g/l, Glucose 4 g/l, Agar 20 g/l pH 6,0) ausgestrichen und 10 - 14 Tage bei 25°C bis zur guten Sporulation inkubiert. Von zwei gut bewachsenen Platten wird eine Sporensuspension (50 ml 0,9 % NaCI; 0,05 % Tween 80) hergestellt und zum Beimpfen von 10 I Produktionsmedium verwendet.

Als Produktionsmedium wird folgende Nährlösung verwendet: 3g/l Rhamnolipid 1 oder 2 oder Alkylester der Rhamnolipide oder Rhamnolipidgemische (z.B. konzentriertes Kulturfiltrat von Pseudomonas aeruginosa, 1 g/l KH₂PO₄, 0,5 g/l (NH₄)₂SO₄, 0,1 g/l MgSO₄ x 7H₂O, 0,1 g/l CaCl₂, 0,1 g/l Casaminoacids pH 5,5. Die Kultur erfolgt im 10 l Blattrührreaktor mit 300 UpM, 0,6 vvm, 27°C bei pH 5,5. Die Kulturdauer beträgt 5 - 10 Tage.

Am Ende der Fermentation werden die Zellen durch Filtration abgetrennt und das Kulturfiltrat steril filtriert (0,22 µm). Dieses Kulturfiltrat enthält den größten Teil (über 90 %) der α-L-Rhamnosidaseaktivität (5000 U/l) und kann direkt oder nach Lyophilisierung bzw. Konzentration durch Ultrafiltration an einer 10 kD Membran zur Spaltung der Rhamnolipide eingesetzt werden.

### Beispiel 5

### Isolierung und Charakterisierung der α-L-Rhamnosidase aus Penicillium sp. DSM 6826

Zur weiteren Reinigung der α-L-Rhamnosidase wurde von einem Konzentrat mit einer Aktivität von 50 U/ml ausgegangen.

Als erster Schritt wird eine Chromatographie an Sepharose Q mit 20mM Tris/HCL pH 7,6 durchgeführt. Die Elution erfolgt mit einem Gradienten von 0-0,5 M NaCl, die erzielte Ausbeute liegt bei ca. 80% bei einem Reinigungsfaktor von 5; das Enzym besitzt eine spez. Aktvität von 62 U/mg Protein.

Diese Fraktion wird weiter über eine Mono P Säule (Pharmacia) chromatographiert (25 mM Imidazol/HCl gegen PBE 74, pH 5,0, 1:12). Die Elution der α-L-Rhamnosidase erfolgt bei einem pH-Wert von 5,6-5,8. Die Ausbeute liegt bei ca. 70%.

Nach entsprechender Umpufferung wird das Protein über eine Superose 12 Säule (1x30 cm) chromatographiert. Als Puffer wird 100 mM Ammoniumacetat pH 5,0 mit 100 mM NaCI verwendet. Die Überprüfung mittels SDS-Gelelektrophorese ergibt eine Proteinbande im Bereich von 60-100 kd abhängig vom Glykosilierungsgrad des Enzyms.

Mit dem gereinigten Enzym wurden die in der folgenden Tabelle zusammengefaßten Untersuchungen durchgeführt.

**Tabelle 3**

| Einfluß verschiedener Substanzen auf die Aktivität der erfindungsgemäßen α-L-Rhamnosidase | |
|---|---|
| Substanz | % Aktivität |
| Kontrolle | 100 |
| CaCl₂ (20 mM) | 127 |
| MgSO₄ (2 mM) | 106 |
| KCl (100 mM) | 69 |
| CsCl (2 mM) | 96 |
| CoCl₂ (2 mM) | 41 |
| CuCl₂ (0,5 mM) | 40 |
| FeSO₄ (0,5 mM) | 100 |
| MnCl₂ (2 mM) | 27 |
| ZnCl₂ (2 mM) | 65 |
| EDTA (10 mM) | 22 |
| EDTA/CaCl2 (10/10 mM) | 90 |
| EGTA (10 mM) | 60 |
| EGTA/MgSO4) (10/10 mM) | 124 |
| L-Rhamnose (0,5 M) | 51 |
| L-Rhamnose (1,0 M) | 39,5 |
| L-Rhamnose (1,5 M) | 26,4 |

### Beispiel 6

### Bestimmung der N-terminalen Sequenz der α-L-Rhamnosidase aus dem Stamm Penicillium sp. DSM 6826 und von Naringinase

Das nach Beispiel 5 gewonnene Enzym aus Penicillium sp. DSM 6826 so wie die nach dem gleichen Verfahren gereinigte Naringinase aus Penicillium decumbens (Rohenzym, Sigma No. N-1385) werden über ein Acrylamidgel (10 %ig) nochmals gereinigt, und nach Transfer der Polypeptide mittels Elektroblotting auf eine ProBlot® -Membran (Fa. Applied Biosystems) übertragen (Sequencers, Nr. 42, April 1990, Applied Biosystems). Die Naringinase fiel dabei als einzelne Bande an, während das gereinigte Enzym aus DSM 6826 sich in zwei sehr eng benachbarte Banden auflösen ließ.
Mittels des Peptid Sequenzers 477a von Applied Biosystems wird die N-terminale Sequenz der drei Polypeptidketten bestimmt. Die Ergebnisse in Tabelle 4 zeigen deutlich, daß beide Polypeptidketten der aktiven Präparation aus dem Stamm DSM 6826 von der Naringinase (Sigmu Nr.-N-1385) verschieden sind.

**Tabelle 4**

| Peptide | N-terminal sequenz |
|---|---|
| Naringinase (96 000 D) | ASVPXGEXILAPSSIELIPT |
| α-L-Rhamnosidase 6826, Peptid I (96 000 D) | DTNDQTSAKVDRGTFDDPAARL |
| α-L-Rhamnosidase 6826, Peptid II (83 500 D) | FFGSX₁QSLYLKLVLKFGTLFD(X₂)A |
| X₁ bedeutet wahrscheinlich Cystein X₂ Aminosäure nicht bestimmt | |

## Patentansprüche

1. Heterogenes Proteingemisch mit der Aktivität einer α-L-Rhamnosidase erhältlich durch
- Fermentation von Penicillium sp. DSM 6825 und/oder 6826,
- Abtrennung von Biomasse von der Kulturbrühe,
- Konzentrierung des Kulturüberstands, wobei das heterogene Proteingemisch eine höhere Spezifität für die Bindungspaltung zwischen terminaler L-Rhamnose und einem Aglykon von rhamnosehaltigen Glykosiden als für die Abspaltung von terminaler Rhamnose aus Flavanonglykosiden hat.

2. Heterogenes Proteingemisch mit der Aktivität einer α-L-Rhamnosidase, mit einem Molekulargewicht von 60 - 100 kd und einem durch Chromatofokussierung ermittelten IEP 5,6 - 5,8, **dadurch gekennzeichnet, daß** das heterogene Proteingemisch mit der Aktivität einer α-L-Rhamnosidase die aminoterminale Aminosäuresequenz D-T-N-D-Q-T-S-A-K-V-D-R-G-T-F-D-D-P-A-A-R-L oder F-F-G-S-X-Q-S-L-Y-L-K-L-V-L-K-F-G-T-L-F-D-X-A beinhaltet und die Spaltung der Bindung zwischen terminaler Rhamnose und Aglykon von rhamnosehaltigen Glykosiden mit einer höheren Spezifität katalysiert als die Abspaltung von terminaler Rhamnose aus Flavanonglykosiden.

3. Heterogenes Proteingemisch mit der Aktivität einer α-L-Rhamnosidase nach Anspruch 2, **dadurch gekennzeichnet, daß** dieses aus Penicillin sp. stammt.

4. Heterogenes Proteingemisch mit der Aktivität einer α-L-Rhamnosidase nach Anspruch 1, **dadurch gekennzeichnet, daß** der durch Chromatofokussierung ermittelte IEP des Enzyms 5,6 - 6,8 ist.

5. Heterogenes Proteingemisch mit der Aktivität einer α-L-Rhamnosidase nach einem oder mehreren der Ansprüche 2 - 4, **dadurch gekennzeichnet, daß** dieses aus Penicillium sp. DSM 6826 stammt.

6. Heterogenes Proteingemisch mit der Aktivität einer α-L-Rhamnosidase nach Anspruch 2, **dadurch gekennzeichnet, daß** das Aglykon eine Fettsäure ist.

7. Heterogenes Proteingemisch mit der Aktivitat einer α-L-Rhamnosidase nach Anspruch 1 oder 2, **dadurch gekennzeichnet, daß** das heterogene Proteingemisch mit der Aktivität einer α-L-Rhamnosidase die Spaltung von Rhamnolipiden katalysiert.

8. Verfahren zur Herstellung eines heterogenen Proteingemisches mit der Aktivität einer α-L-Rhamnosidase nach Anspruch 1, **dadurch gekennzeichnet, daß** Penicillium sp. in einem Nährmedium kultiviert wird, bis sich ein heterogenes Proteingemisch mit der Aktivität einer α-L-Rhamnosidase in der Kultur anhäuft, die Biomasse von der Kulturbrühe abgetrennt und der so erhaltene Kulturüberstand aufkonzentriert wird.

9. Verfahren nach Anspruch 8, **dadurch gekennzeichnet, daß** das Nährmedium als Kohlenstoffquelle Rhamnolipide und/oder Rhamnolipidderivate enthält.

10. Verfahren nach Anspruch 8, **dadurch gekennzeichnet, daß** das Penicillium sp. DSM 6825 und/oder 6826 kultiviert werden.

11. Verwendung eines heterogenen Proteingemisches mit der Aktivität einer α-L-Rhamnosidase nach Anspruch 1 oder 2 zur Herstellung von L-Rhamnose.

12. Penicillium sp. DSM 6825.

13. Penicillium sp. DSM 6826.

## Claims

1. A heterogeneous protein mixture having the activity of an α-L-rhamnosidase, which can be obtained by
- fermenting Penicillium sp. DSM 6825 and/or 6826
- separating off the biomass from the culture broth,
- concentrating the culture supernatant, with the heterogeneous protein mixture having a higher specificity for the cleavage of the bond between terminal L-rhamnose and an aglycone of rhamnose-containing glycosides than for the elimination of terminal rhamnose from flavanone glycosides.

2. A heterogeneous protein mixture having the activity of an α-L-rhamnosidase, having a molecular weight of 60-100 kd and an IEP, ascertained by chromatofocussing, of 5.6-5.8, which heterogeneous protein mixture having the activity of an α-L-rhamnosidase contains the amino-terminal amino acid sequence D-T-N-D-Q-T-S-A-K-V-D-R-G-T-F-D-D-P-A-A-R-L or F-F-G-S-X-Q-S-L-Y-L-K-L-V-L-K-F-G-T-L-F-D-X-A and catalyzes the cleavage of the bond between terminal rhamnose and the aglycone of rhamnose-containing glycosides at a higher specificity than the elimination of terminal rhamnose from flavanone glycosides.

3. The heterogeneous protein mixture having the activity of an α-L-rhamnosidase as claimed in claim 2, wherein the mixture originates from Penicillium sp.

4. The heterogeneous protein mixture having the activity of an α-L-rhamnosidase as claimed in claim 1, wherein the IEP of the enzyme, ascertained by chromatofocussing, is 5.6-6.8.

5. The heterogeneous protein mixture having the activity of an α-L-rhamnosidase as claimed in one or more of claims 2-4, wherein the mixuture originates from Penicillium sp. DSM 6826.

6. The heterogeneous protein mixture having the activity of an α-L-rhamnosidase as claimed in claim 2, wherein the aglycone is a fatty acid.

7. The heterogeneous protein mixture having the activity of an α-L-rhamnosidase as claimed in claim 1 or 2, which heterogeneous protein mixture having the activity of an α-L-rhamnosidase catalyzes the cleavage of rhamnolipids.

8. A process for preparing a heterogeneous protein mixture having the activity of an α-L-rhamnosidase as claimed in claim 1, wherein Penicillium sp. is cultivated in a nutrient medium until a heterogeneous protein mixture having the activity of an α-L-rhamnosidase accumulates in the culture, the biomass is separated off from the culture broth, and the culture supernatant thus obtained is concentrated.

9. The process as claimed in claim 8, wherein the nutrient medium contains rhamnolipids and/or rhamnolipid derivatives as the carbon source.

10. The process as claimed in claim 8, wherein Penicillium sp. DSM 6825 and/or 6826 are cultivated.

11. Use of a heterogeneous protein mixture having the activity of an α-L-rhamnosidase as claimed in claim 1 or 2 for the preparation of L-rhamnose.

12. Penicillium sp. DSM 6825.

13. Penicillium sp. DSM 6826.

## Revendications

1. Mélange hétérogène de protéines présentant l'activité d'une α-L-rhamnosidase, qui peut être obtenu par
- fermentation de Penicillium sp. DSM 6825 et/ou 6826,
- séparation de la biomasse du bouillon de culture,
- concentration du surnageant de culture, le mélange hétérogène de protéines présentant une spécificité plus élevée pour le clivage de la liaison entre le L-rhamnose terminal et une aglucone de glucosides contenant du rhamnose que pour le clivage du rhamnose terminal à partir de glucosides flavanoniques.

2. Mélange hétérogène de protéines présentant l'activité d'une α-L-rhamnosidase, avec une masse molaire de 60 à 100 kd et un point isoélectrique (IEP) de 5,6 à 5,8, déterminé par chromatofocalisation, **caractérisé en ce que** le mélange hétérogène de protéines présentant l'activité d'une α-L-rhamnosidase contient la séquence aminoterminale d'acides aminés D-T-N-D-Q-T-S-A-K-V-D-R-G-T-F-D-D-P-A-A-R-L ou F-F-G-S-X-Q-S-L-Y-L-K-L-V-L-K-F-G-T-L-F-D-X-A et catalyse, avec une spécificité plus élevée, le clivage de la liaison entre le rhamnose terminal et l'aglucone de glucosides contenant du rhamnose que le clivage du rhamnose terminal de glucosides flavanoniques.

3. Mélange hétérogène de protéines présentant l'activité d'une α-L-rhamnosidase selon la revendication 2, **caractérisé en ce que** celui-ci provient de Pénicilline sp.

4. Mélange hétérogène de protéines présentant l'activité d'une α-L-rhamnosidase selon la revendication 1, **caractérisé en ce que** l'IEP de l'enzyme déterminé par chromatofocalisation est de 5,6 à 6,8.

5. Mélange hétérogène de protéines présentant l'activité d'une α-L-rhamnosidase selon une ou plusieurs des revendications 2 à 4, **caractérisé en ce que** celui-ci provient de Penicillium sp. DSM 6826.

6. Mélange hétérogène de protéines présentant l'activité d'une α-L-rhamnosidase selon la revendication 2, **caractérisé en ce que** l'aglucone est un acide gras.

7. Mélange hétérogène de protéines présentant l'activité d'une α-L-rhamnosidase selon la revendication 1 ou 2, **caractérisé en ce que** le mélange hétérogène de protéines présentant l'activité d'une α-L-rhamnosidase catalyse le clivage de rhamnolipides.

8. Procédé pour la préparation d'un mélange hétérogène de protéines présentant l'activité d'une α-L-rhamnosidase selon la revendication 1, **caractérisé en ce que** Penicillium sp. est cultivé dans un milieu nutritif jusqu'à ce qu'un mélange hétérogène de protéines présentant l'activité d'une α-L-rhamnosidase s'accumule dans la culture, la biomasse est séparée du bouillon de culture et le surnageant de culture ainsi obtenu est concentré.

9. Procédé selon la revendication 8, **caractérisé en ce que** le milieu nutritif contient, en tant que source de carbone, des rhamnolipides et/ou des dérivés de rhamnolipides.

10. Procédé selon la revendication 8, **caractérisé en ce que** le Penicillium sp. DSM 6825 et/ou 6826 sont cultivés.

11. Utilisation d'un mélange hétérogène de protéines présentant l'activité d'une α-L-rhamnosidase selon la revendication 1 ou 2, pour la préparation de L-rhamnose.

12. Penicillium sp. DSM 6825.

13. Penicillium sp. DSM 6826.
